# NEUE EUROPÄISCHE PATENTSCHRIFT

(11) **EP 0 652 421 B2**
(45) Veröffentlichungstag und Bekanntmachung des Hinweises auf die Entscheidung über den Einspruch: **04.06.2003**
(45) Hinweis auf die Patenterteilung: 20.05.1998
(21) Anmeldenummer: 94116976.5
(22) Anmeldetag: 27.10.1994
(51) Int. Cl.: G01F 11/02

(54) **Dispenser**
Dispenser
Dispenseur

(30) Priorität: 04.11.1993 DE 9316886 U
(43) Veröffentlichungstag der Anmeldung: 10.05.1995
(73) Patentinhaber: BRAND GMBH + CO KG, 97877 Wertheim (DE)
(72) Erfinder: Böhm, Dieter, D-36391 Sinntal (DE); Krank, Peter, D-97877 Wertheim (DE); Wanner, Jürgen, D-97877 Wertheim (DE)
(74) Vertreter: Gesthuysen, von Rohr & Eggert

(56) Entgegenhaltungen:
- EP-A- 0 542 241
- DE-A- 3 019 426
- DE-A- 3 343 134
- DE-A- 3 504 107
- DE-C- 3 800 667
- GB-A- 386 410
- US-A- 4 306 670

## Beschreibung

Die Erfindung betrifft einen Dispenser zum Ausgeben einer Flüssigkeit aus einem Behälter mit den Merkmalen des Oberbegriffs von Anspruch 1.

Ein solcher Dispenser ist aus der EP-A-0 542 241 bekannt. Er hat eine Rückleitung, die stromab des Druckventils von der Druckleitung abzweigt. Bei Flüssigkeitsförderung über die Rückleitung muß die Flüssigkeit daher das selbsttätige Druckventil passieren.

Selbsttätige, durch das durchströmende Medium gesteuerte Ventile können bei bestimmten Medien, z.B. zum Auskristallisieren neigenden Flüssigkeiten, verkleben. In einem Dispenser der genannten Art können sich dann statische Drucke von einigen bar Überdruck aufbauen. Da der Dispenser nicht auf eine solche Druckbelastung ausgelegt ist, besteht die Gefahr eines unkontrollierten Flüssigkeitsaustritts.

Falls bei dem Dispenser nach der EP - A - 0 542 241 das Druckventil im Betrieb verklebt und verstopft, hat der Benutzer nach dem Ansaugen von Flüssigkeit in den Dispenser keine Möglichkeit mehr, die Flüssigkeit über die Rückleitung in den Behälter zurückzugeben und den Dispenser so vor einer möglichen Reinigung insbesondere des verklebten Ventils zu entleeren. Die für die Reinigung erforderliche Handhabung des flüssigkeitsgefüllten Dispensers ist im Fall unsachgemäßer Behandlung insbesondere bei gefährlichen Medien mit möglichen Gefahren für den Anwender verbunden, zu deren Abwehr der Anwender Vorkehrungen treffen muß, wie z. B. Abnehmen des Dispensers vom Behälter, u. U. Transport des flüssigkeitsgefüllten Dispensers in einem geeigneten Transportbehälter, Reinigung des Dispensers an einem Arbeitsplatz, der für den zwangsläufig auftretenden Austritt von Medium aus dem gefüllten Dispenser ausgestattet ist, Schutzmaßnahmen zur Vermeidung von Kontamination der Person, die den Dispenser reinigt, u. a. m.

Aufgabe der Erfindung ist es, die Betriebssicherheit eines Dispensers der eingangs genannten Art zu verbessern.

Diese Aufgabe wird mit einem gattungsgemäßen Dispenser gelöst, bei dem die Rückleitung vor dem Druckventil von der Druckleitung abgeht.

Erfindungsgemäß muß bei Flüssigkeitsförderung über die Rückleitung die Flüssigkeit nicht das Druckventil passieren. Falls das Druckventil im Betrieb verkleben sollte, besteht so die Möglichkeit, den Dispenser über die Rückleitung zurück in den Behälter zu entleeren. Dadurch wird die Kontaminationsgefahr für die Person, die den Dispenser reinigt, erheblich verringert. Auch kann der Aufwand für die Wartung des Dispensers erheblich reduziert werden.

Vorzugsweise läßt sich die Rückleitung des erfindungsgemäßen Dispensers durch ein darin liegendes Ventil wahlweise absperren und freigeben. Das Ventil ist vorzugsweise gesteuert. Es kann aber auch ein selbsttätiges Ventil sein.

In der Druckleitung des erfindungsgemäßen Dispensers liegt vorzugsweise ein Wegeventil, das die Druckleitung in einer Schaltstellung sperrt und in einer anderen Schaltstellung freigibt. Das Wegeventil kann ein 2/2-Wegeventil oder 3/2-Wegeventil sein und vor oder hinter dem Druckventil liegen.

In der Rückleitung liegt ferner vorzugsweise ein Rückschlagventil, das sich flüssigkeitsdruckbetätigt hin zu dem Behälter öffnet. Das Rückschlagventil verhindert ein Ansaugen von Luft durch die Rückleitung.

In der Rückleitung kann des weiteren ein gesteuertes Wegeventil liegen, das die Rückleitung in einer Schaltstellung sperrt und in einer anderen Schaltstellung freigibt. Ein solches Wegeventil ist für die Erfindung allerdings nicht zwingend. Vielmehr kann der Strömungsweg über die Rückleitung auch allein durch das selbsttätige Rückschlagventil freigegeben werden. Letzteres ist dazu vorzugsweise so ausgelegt, daß sein Öffnungsbetätigungsdruck höher ist als der des Druckventils.

Ebenso wie in der Druckleitung kann in der Rückleitung des erfindungsgemäßen Dispensers ein 2/2-Wegeventil liegen. Bei einer bevorzugten Ausführungsform liegt in der Druckleitung ein 3/2-Wegeventil, das in der einen Schaltstellung die Druckleitung freigibt und die Rückleitung sperrt und in der anderen Schaltstellung die Druckleitung sperrt und die Rückleitung freigibt.

Die Erfindung wird im folgenden anhand von Ausführungsbeispielen näher erläutert.
Es zeigen:
- Fig. 1: bis Fig. 5 fluidische Schaltungen erfindungsgemäßer Dispenser;
- Fig. 6: die Schnittansicht eines Dispensers nach der Schaltung Fig. 3; und
- Fig. 7: die Schnittansicht eines Dispensers nach der Schaltung Fig. 5.

Die Dispenser haben eine Dosierpumpe in Form einer Kolbenpumpe mit einem einfachwirkenden Tauchkolben 10, der unter Abdichtung in einer Zylinderlaufbuchse 12 angeordnet ist und mit letzterer einen Arbeitsraum 14 begrenzt. An den Arbeitsraum 14 führt eine Anschlußleitung 16, die sich in eine Saugleitung 18 und eine Druckleitung 20 verzweigt. Die Saugleitung 18 führt an den Boden eines Behälters 22, auf den der Dispenser aufgesetzt ist. Die Druckleitung 20 führt zu einem Auslaß 24 für in dosierter Menge aus dem Behälter 22 gepumpte Flüssigkeit.

In der Saugleitung 18 liegt ein Saugventil 26, das aus einem selbsttätigen, zu dem Behälter 22 hin schließenden Rückschlagventil besteht. In der Druckleitung 20 liegt ein Druckventil 28, das als Ausstoßventil dient und aus einem selbsttätigen, zu der Kolbenpumpe hin schließenden Rückschlagventil besteht.

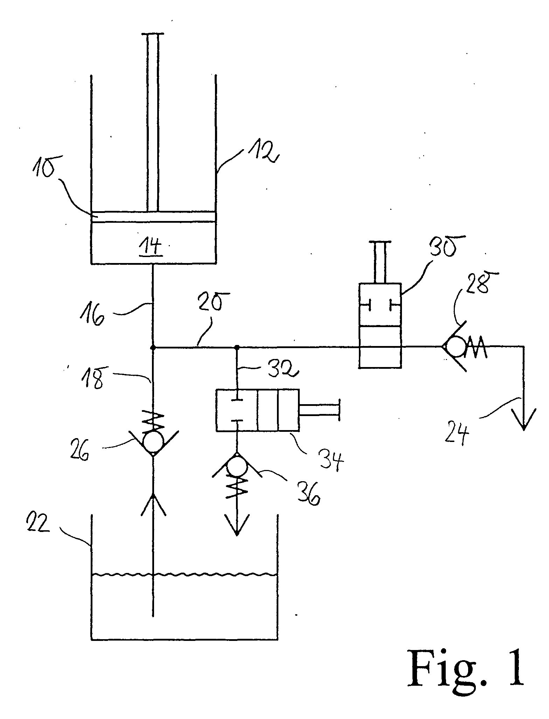

Gemäß Fig. 1 liegt in der Druckleitung 20 vor dem Druckventil 28 ein gesteuertes 2/2-Wegeventil 30, das die Druckleitung 20 in der einen Schaltstellung freigibt und in der anderen Schaltstellung sperrt. Noch vor dem 2/2-Wegeventil 30 zweigt von der Druckleitung 20 eine Rückleitung 32 ab, die in den Behälter 22 zurückführt. In der Rückleitung 32 liegen in Strömungsrichtung hintereinander ein gesteuertes 2/2-Wegeventil 34 und ein selbsttätiges Rückschlagventil 36, das zu der Kolbenpumpe hin schließt. Das 2/2-Wegeventil 34 gibt die Rückleitung 32 in der einen Schaltstellung frei und sperrt sie in der anderen Schaltstellung.

Im normalen Dosierförderbetrieb befindet sich das Druckleitungs-Wegeventil 30 in Durchgangsstellung und das Rückleitungs-Wegeventil 36 in Sperrstellung. Im Saughub der Kolbenpumpe wird Flüssigkeit über die Saugleitung 18 aus dem Behälter 22 angesaugt und in Druckhub hin zu dem Auslaß 24 gefördert.

Im Rücklaufbetrieb befindet sich das Druckleitungs-Wegeventil 30 in Sperrstellung und das Rückleitungs-Wegeventil 36 in Durchgangsstellung. Im Saughub der Kolbenpumpe angesaugte Flüssigkeit wird im Druckhub über die Rückleitung 32 zurück in den Behälter 22 gefördert. Der Dispenser kann so durch Umpumpen entlüftet werden. Ebenso lassen sich die Kolbenpumpe und die Druckleitung 20 durch die Rückleitung 32 entleeren. Das in der Rückleitung 32 liegende Rückschlagventil 36 verhindert, daß im Saughub über die Rückleitung 32 Luft angesaugt wird.

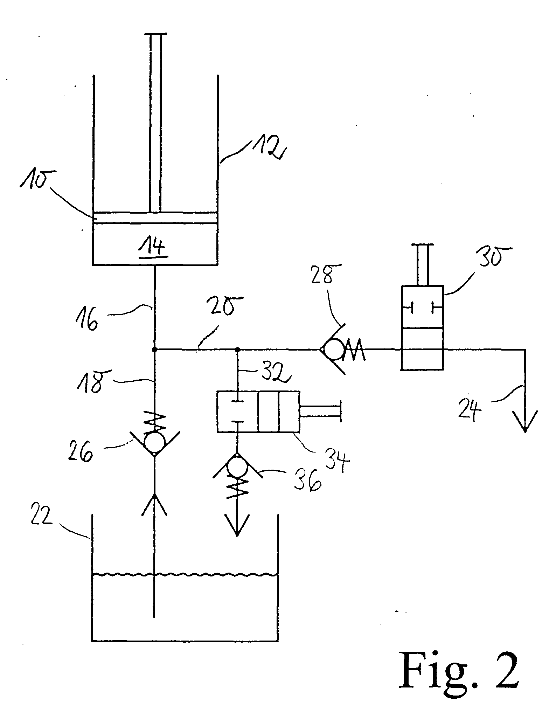

In Fig. 2 ist verglichen mit Fig. 1 die Reihenfolge von Druckventil 28 und 2/2-Wegeventil 30 in der Druckleitung 20 vertauscht. Die Funktion ist die gleiche.

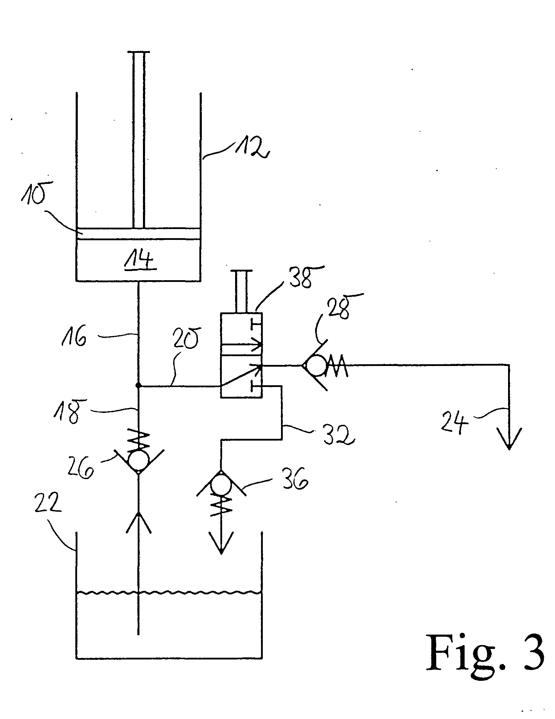

In Fig. 3 sind verglichen mit Fig. 2 die beiden 2/2-Wegeventile 30, 34 durch ein gesteuertes 3/2-Wegeventil 38 ersetzt, das in der einen, dem Dosierförderbetrieb zugeordneten Schaltstellung die Druckleitung 20 freigibt und die Rückleitung 32 sperrt und in der anderen, dem Rücklaufbetrieb zugeordneten Schaltstellung die Druckleitung 20 sperrt und die Rückleitung 32 freigibt.

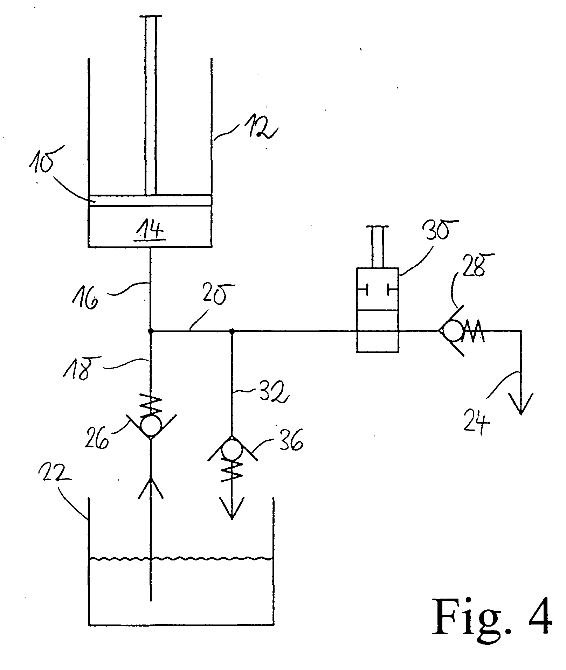

In Fig. 4 ist verglichen mit Fig. 2 das 2/2-Wegeventil 34 in der Rückleitung 32 entfallen. Die Rückleitung 32 wird allein von dem Rückschlagventil 36 beherrscht, dessen Öffnungsbetätigungsdruck höher ist als der des Druckventils 28. In der dem Dosierförderbetrieb zugeordneten Durchgangsstellung des 2/2-Wegeventils 30 in der Druckleitung 20 wird Flüssigkeit allein über das Druckventil 28 hin zum Auslaß 24 gefördert, da sich in der Druckleitung 20 kein zum Öffnen des Rückschlagventils 36 ausreichender Betriebsdruck aufbauen kann. In der dem Rücklaufbetrieb zugeordneten Sperrstellung des 2/2-Wegeventils 30 ist das hingegen möglich.

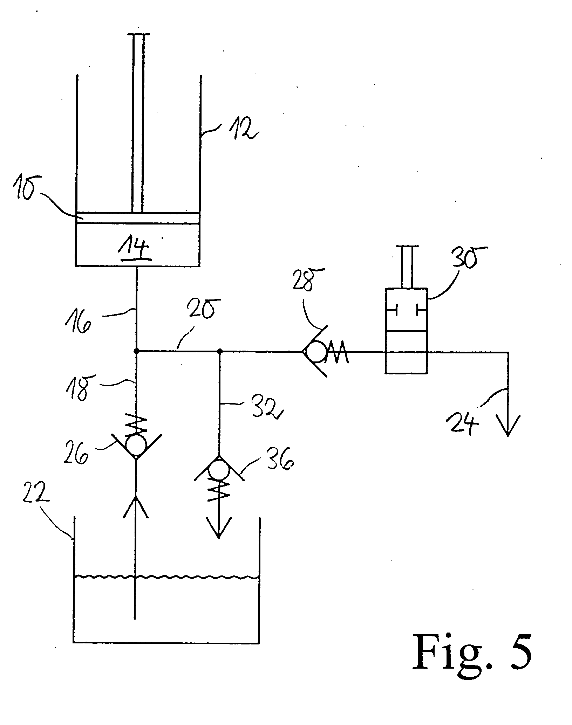

In Fig. 5 ist verglichen mit Fig. 4 die Reihenfolge von Druckventil 28 und 2/2-Wegeventil 30 in der Druckleitung 20 vertauscht. Die Funktion ist die gleiche.

Alle Rückschlagventile 26, 28, 36 einschließlich der Saugventile 26 und Druckventile 28 können sowohl federbetätigt, als auch schwerkraftbetätigt sein. Alle gesteuerten Ventile 30, 34, 38 können handbetätigt, elektrisch, hydraulisch oder pneumatisch betätigt sein, in allen oder einem Teil ihrer Schaltstellungen einrasten und/oder durch Federkraft in bestimmte Schaltstellungen vorgespannt sein.

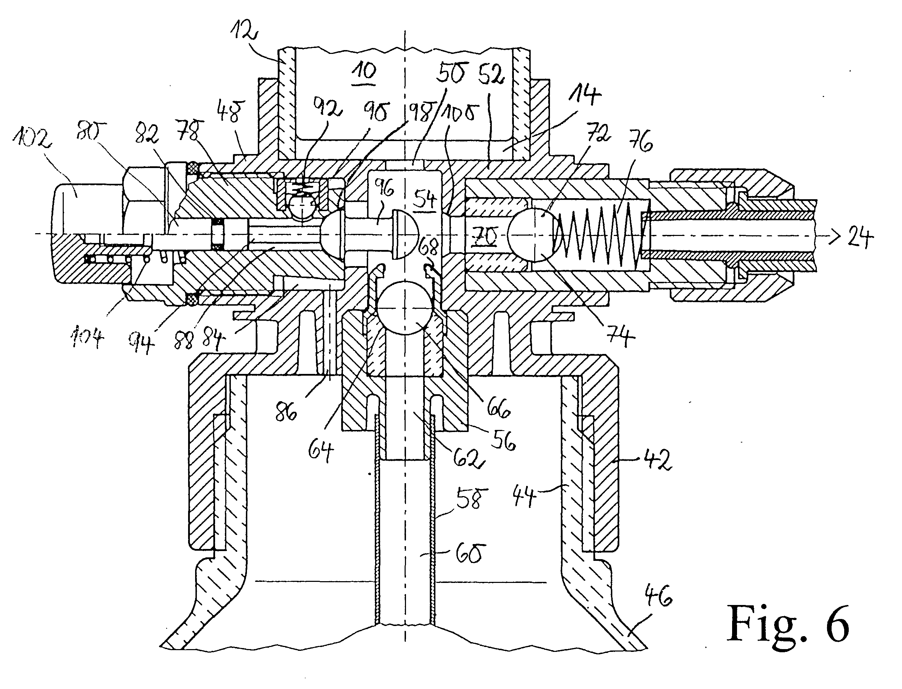

Fig. 6 zeigt einen Flaschendispenser nach der Schaltung Fig. 3. Der Dispenser sitzt mit einem zylindrischen Überwurf 42 auf dem Hals 44 einer Flasche 46, die eine dosiert auszugebende Flüssigkeit enthält. Der Dispenser hat ein Ventilgehäuse 48, das im aufgesetzten Zustand oberhalb der Flaschenöffnung zu liegen kommt. Auf das Ventilgehäuse 48 baut in mittiger, axialer Anordnung eine Kolbenpumpe mit einer Zylinderlaufbuchse 12 und einem Kolben 10 auf. Der Kolben 10 ist ein einfachwirkender Tauchkolben. Er begrenzt mit der Zylinderlaufbuchse 12 einen Arbeitsraum 14. Der Arbeitsraum 14 steht über eine mittige Öffnung 50 im Zylinderboden 52 mit einer Ventilkammer 54 des Ventilgehäuses 48 in Verbindung.

Von einem unteren Ventileinsatz 56 des Ventilgehäuses 48 geht in mittiger, axialer Anordnung ein Tauchrohr 58 ab, das nach unten in die Flasche 46 ragt und dicht über ihrem Boden endet. Das Innere 60 des Tauchrohrs 58 steht über einen mittigen, axialen Saugkanal 62 des Ventileinsatzes 56 und ein darin liegendes Rückschlagventil mit der Ventilkammer 54 in Verbindung. Das Rückschlagventil dient als Saugventil 26 der Kolbenpumpe. Es ist schwerkraftbetätigt. Zu dem Saugventil 26 gehört ein an dem Ventileinsatz 56 befindlicher Ventileinsatz 64 und eine darauf ruhende Ventilkugel 66, die zwischen Halteklauen 68 gefangen ist und den Strömungsweg durch das Tauchrohr 58 normalerweise sperrt. Die Ventilkugel 66 wird im Saughub der Kolbenpumpe von dem Ventilsitz 64 abgehoben und letztgenannter Strömungsweg wird freigegeben.

Die Ventilkammer 54 hat einen radialen Abgang 70, der zu einem Auslaß 24 des Dispensers führt. Vor dem Abgang 70 liegt ein als Druckventil 28 der Kolbenpumpe dienendes Rückschlagventil. Das Druckventil 28 hat einen Ventilsitz 72 und eine federbeaufschlagte Ventilkugel 74, die den Abgang 70 normalerweise sperrt. Die Ventilkugel 74 wird im Druckhub der Kolbenpumpe gegen die Kraft der Ventilfeder 76 von ihrem Ventilsitz 72 abgehoben und der Strömungsweg hin zu dem Auslaß 24 freigegeben. Auf der dem Abgang 70 gegenüberliegenden Seite der Ventilkammer 54 ist ein Ventileinsatz in das Ventilgehäuse 48 eingelassen. Der Ventileinsatz besteht aus einer zylindrischen Führungsbuchse 78 und einem mittig darin aufgenommenen Ventilschieber 80, der gegen die Führungsbuchse 78 abgedichtet 82 und in Längsrichtung der Führungsbuchse, d.h. quer zu der Achse der Flasche 46 verschieblich ist.

Das in das Ventilgehäuse 48 hineinragende Ende der Führungsbuchse 78 hat eine Durchmesserabstufung, durch die die Führungsbuchse 78 mit dem Ventilgehäuse 48 einen Ringraum 84 begrenzt. Von dem Ringraum 84 führt eine Axialbohrung 86 nach unten in die Flasche 46. Die Führungsbuchse 78 hat eine Radialbohrung, die die zur Aufnahme des Ventilschiebers 80 dienende Mittelbohrung 88 mit dem Ringraum 84 verbindet. In der Radialbohrung liegt ein Rückschlagventil 36 mit einem Ventilsitz und einer Ventilkugel 90. Letztere ist durch eine Ventilfeder 92 in eine Schließstellung vorgespannt, in der sie den Strömungsweg durch die Radialbohrung sperrt.

Der Ventilschieber 80 ist auf Höhe des Rückschlagventils 36 unter Bildung eines Schafts 94 kleineren Durchmessers abgesetzt. Am inneren Ende des Schafts 94 setzt ein diaboloförmiges Ventilglied 96 an, das in der Ventilkammer 54 aufgenommen ist und wahlweise Dichtstellungen an einander diametral gegenüberliegenden Ventilsitzen 98, 100 einnimmt.

Der Ventilschieber 80 ist mit einem aus dem seitlichen Ventileinsatz herausragenden Betätigungsknopf 102 verbunden. Er ist durch eine Druckfeder 104 in eine Ausfahrstellung vorgespannt, in der das Ventilglied 96 mit einem vor der Mittelbohrung 88 der Führungsbuchse 78 liegenden Ventilsitz 98 zusammenwirkt. Durch Hineindrücken des Betätigungsknopfes 102 gegen die Kraft der Druckfeder 104 hebt das Ventilglied 96 von diesem Ventilsitz 98 ab, und es gelangt in eine Dichtstellung an dem gegenüberliegenden Ventilsitz 100 vor dem Abgang 70 hin zu dem Auslaß 24.

Die in Fig. 6 dargestellte Ventilstellung mit ausgefahrenem Betätigungsknopf 102 entspricht dem Dosierförderbetrieb. Für den Rücklaufbetrieb wird der Betätigungsknopf 102 gedrückt und niedergehalten. Dadurch wird der Strömungsweg hin zu dem Auslaß 24 gesperrt und eine Rückleitung 32 über die Mittelbohrung 88 der Führungsbuchse 78, das Rückschlagventil 36, den Ringraum 84 und die Axialbohrung 86 eröffnet. Das Rückschlagventil 36 wird gegen die Kraft der Ventilfeder 92 durch den im Druckhub der Kolbenpumpe anliegenden Flüssigkeitsdruck geöffnet. Es schließt im Saughub der Kolbenpumpe und verhindert, daß über die Rückleitung 36 Luft aus der Flasche 46 angesaugt wird.

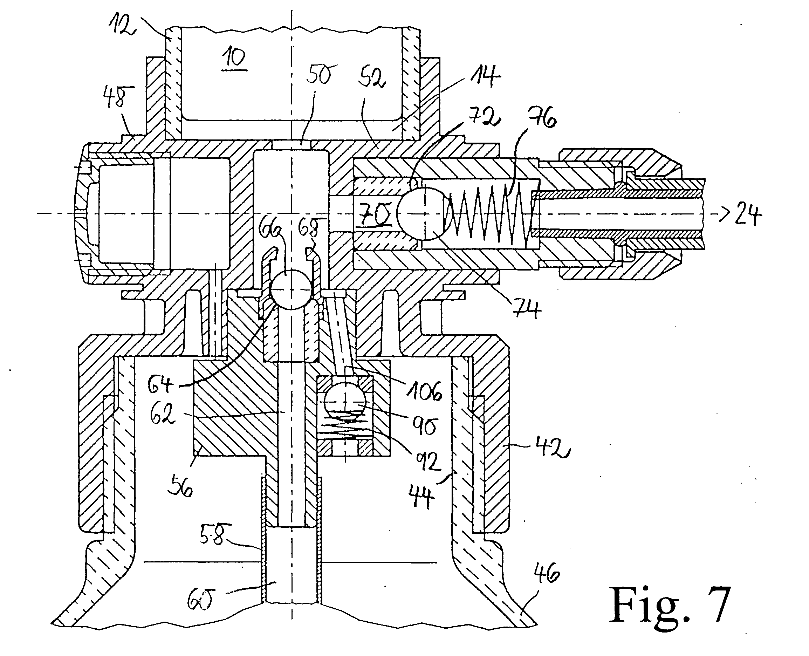

Fig. 7 zeigt einen Flaschendispenser nach der Schaltung Fig. 5. Hinsichtlich der Anordnung und Gestaltung von Überwurf 42, Ventilgehäuse 48, Kolbenpumpe, Tauchrohr 58, Abgang 70 hin zu dem Auslaß 24, Saugventil 26 und Druckventil 28 gelten die vorstehenden Ausführungen entsprechend.

Der untere Ventileinsatz 56 des Dispensers ist neben dem Saugkanal 62 durchbohrt und in einer Erweiterung der Bohrung 106 ein Rückschlagventil 36 angeordnet. Das Rückschlagventil 36 ist federbetätigt. Es hat einen Ventileinsatz und eine Ventilkugel 90, die durch eine Ventilfeder 92 in eine die Bohrung 106 sperrende Schließstellung vorgespannt ist.

Die Ventilkugel 90 wird im Druckhub der Kolbenpumpe gegen die Kraft der Ventilfeder 92 von ihrem Dichtsitz abgehoben, so daß eine Rückleitung von Flüssigkeit durch die Bohrung 106 stattfindet. Die Ventilfedern 76, 92 des Druckventils 28 und des Rückschlagventils 36 sind so aufeinander abgestimmt, daß im Dosierförderbetrieb nur das Druckventil 28, nicht aber das Rückschlagventil 36 öffnet. Um einen zum Öffnen des Rückschlagventils 36 ausreichenden Druckaufbau zu erreichen, muß stromab von dem Druckventil 28 ein nicht näher dargestelltes Sperrventil geschlossen werden.

Auf der dem Abgang 70 hin zu dem Auslauf 24 gegenüberliegenden Seite hat der Dispenser eine zur Belüftung der Flasche 46 dienende Baugruppe. Diese ist bei dem Dispenser gemäß Fig. 6 mit im übrigen geringen baulichen Modifikationen durch den seitlichen Ventilschiebereinsatz ersetzt.

## Patentansprüche

1. Dispenser zum Ausgeben einer Flüssigkeit aus einem Behälter (22)
mit einer Pumpe (10, 12, 14),
mit einer Saugleitung (18), die aus dem Behälter (22) zu der Pumpe (10, 12, 14) führt und in der ein selbsttätiges Saugventil (26) liegt,
mit einer Druckleitung (20), die von der Pumpe (12) zu einem Auslaß (24) führt und in der ein selbsttätiges Druckventil (28) liegt, und
mit einer durch Betätigungseingriff eröffenbaren Rückleitung (32) von der Druckleitung (20) in den Behälter (22) zurück,
**dadurch gekennzeichnet,**
**daß** die Rückleitung (32) vor dem Druckventil (28) von der Druckleitung (20) abgeht.

2. Dispenser nach Anspruch 1, **dadurch gekennzeichnet, daß** die Rückleitung (32) durch ein darin liegendes Ventil (34; 36) wahlweise absperrbar und freigebbar ist.

3. Dispenser nach Anspruch 2, **dadurch gekennzeichnet, daß** das Ventil (36) in der Rückleitung (32) selbsttätig ist.

4. Dispenser nach einem der Ansprüche 1 bis 3, **dadurch gekennzeichnet, daß** in der Rückleitung (32) ein Rückschlagventil (36) liegt, das sich flüssigkeitsdruckbetätigt zu dem Behälter (22) hin öffnet.

5. Dispenser nach Anspruch 4, **dadurch gekennzeichnet, daß** der Öffnungsbetätigungsdruck des Rückschlagventils (36) höher ist als der Öffnungsbetätigungsdruck des Druckventils (28).

6. Dispenser nach einem der Ansprüche 1 bis 5, **dadurch gekennzeichnet, daß** in der Rückleitung (32) ein gesteuertes Wegeventil (34) liegt, das die Rückleitung (32) in einer Schaltstellung sperrt und in einer anderen Schaltstellung freigibt.

7. Dispenser nach Anspruch 6, **dadurch gekennzeichnet, daß** das Wegeventil (34) in der Rückleitung (32) ein 2/2-Wegeventil ist.

8. Dispenser nach einem der Ansprüche 1 bis 7, **dadurch gekennzeichnet, daß** in der Druckleitung (20) ein gesteuertes Wegeventil (30; 38) liegt, das die Druckleitung (20) in einer Schaltstellung sperrt und in einer anderen Schaltstellung freigibt.

9. Dispenser nach Anspruch 8, **dadurch gekennzeichnet, daß** das Wegeventil in der Druckleitung (20) ein 2/2-Wegeventil (30) oder ein 3/2-Wegeventil (38) ist.

10. Dispenser nach Anspruch 8 oder 9, **dadurch gekennzeichnet, daß** das Wegeventil (30; 38) in der Druckleitung (20) vor dem Druckventil (28) liegt.

11. Dispenser nach Anspruch 8 oder 9, **dadurch gekennzeichnet, daß** das Wegeventil (30; 38) in der Druckleitung (20) hinter dem Druckventil (28) liegt.

12. Dispenser nach den Ansprüchen 6 und 10, **dadurch gekennzeichnet, daß** in der Druckleitung (20) ein 3/2-Wegeventil (38) liegt, das in der einen Schaltstellung die Druckleitung (20) freigibt und die Rückleitung (32) sperrt und in der anderen Schaltstellung die Druckleitung (20) sperrt und die Rückleitung (32) freigibt.

## Claims

1. A dispenser for dispensing fluid from a container (22), with a pump (10,12,14), with a suction line (18) which leads from the container (22) to the pump (10, 12, 14) and in which an automatic suction valve (26) is disposed, with a pressure line (20) which leads from the pump (12) to an outlet (24) and in which an automatic pressure valve (28) is disposed, and with a return line (32) from the pressure line (20) back into the container, which can be opened by actuating engagement, **characterised in that** the return line (32) starts from the pressure line (20) upstream of the pressure valve (28).

2. A dispenser according to Claim 1, **characterised in that** the return line (32) can be alternately shut off and opened by a valve (34,36) disposed therein.

3. A dispenser according to Claim 2, **characterised in that** the valve (36) in the return line (32) is automatic.

4. A dispenser according to any one of Claims 1 to 3, **characterised in that** a non-return valve (36) is disposed in the return line (32), which opens towards the container (22) actuated by hydraulic pressure.

5. A dispenser according to Claim 4, **characterised in that** the opening actuating pressure of the non-return valve (36) is higher than the opening actuating pressure of the pressure valve (28).

6. A dispenser according to any one of Claims 1 to 5, **characterised in that** a controlled directional valve (34) is disposed in the return line (32), which shuts off the return line (32) in one operating position and opens it in another operating position.

7. A dispenser according to Claim 6, **characterised in that** the directional valve in the return line (32) is a 2/2-way valve.

8. A dispenser according to any one of Claims 1 to 7, **characterised in that** a controlled directional valve (30; 38) is disposed in the pressure line (20), which shuts off the pressure line (20) in one operating position and opens its in another operating position.

9. A dispenser according to Claim 8, **characterised in that** the directional valve in the pressure line (20) is a 2/2-way valve (30) or a 3/2-way valve (38).

10. A dispenser according to Claim 8 or 9, **characterised in that** the directional valve (30;38) in the pressure line (20) is disposed upstream of the pressure valve (28).

11. A dispenser according to Claim 8 or 9, **characterised in that** the directional valve (30; 38) in the pressure line (20) is disposed downstream of the pressure valve (28).

12. A dispenser according to Claims 6 and 10, **characterised in that** a 3/2-way valve (38) is disposed in the pressure line (20), which in one operating position opens the pressure line (20) and shuts off the return line (32) and in the other operating position shuts off the pressure line (20) and opens the return line (32).+

## Revendications

1. Distributeur pour la distribution d'un liquide à partir d'un réservoir (22), comportant une pompe (10, 12, 14), comportant une conduite d'aspiration (18), qui mène du réservoir (22) à la pompe (10, 12, 14) et dans laquelle est prévue une soupape d'aspiration (26) automatique, comportant une conduite sous pression (20), qui mène de la pompe (12) à une sortie (24) et dans laquelle est montée une soupape de refoulement (28) automatique, et comportant une conduite de retour (32), menant de la conduite de refoulement (20) au réservoir (22), conduite pouvant être ouverte par intervention d'un actionnement, **caractérisé en ce que** la conduite de retour (32) part de la conduite de refoulement (20), devant la soupape de refoulement (28).

2. Distributeur selon la revendication 1, **caractérisé en ce que** la conduite de retour (32) peut être fermée et ouverte au choix par une soupape (34; 36) se trouvant à l'intérieur

3. Distributeur selon la revendication 2, **caractérisé en ce que** la soupape (36) dans la conduite de retour (32) est automatique.

4. Distributeur selon l'une des revendications 1 à 3, **caractérisé en ce que** dans la conduite de retour (32) est prévu un clapet anti-retour (36), qui s'ouvre, de manière actionnée par la pression du liquide, vers le réservoir (22).

5. Distributeur selon la revendication 4, **caractérisé en ce que** la pression d'actionnement pour l'ouverture du clapet anti-retour (36) est supérieure à celle de la soupape de refoulement (28).

6. Distributeur selon l'une des revendications 1 à 5, **caractérisé en ce que** dans la conduite de retour (32) est prévue une soupape de distribution (34) commandée, qui ferme la conduite de retour (32) dans une position de commutation et qui l'ouvre dans une autre position de commutation.

7. Distributeur selon la revendication 6, **caractérisé en ce que** la soupape de distribution (34) prévue dans la conduite de retour (32) est une soupape à 2/2 voies.

8. Distributeur selon l'une des revendications 1 à 7, **caractérisé en ce que** dans la conduite de refoulement (20) est prévue une soupape de distribution (30; 38) commandée, qui ferme la conduite de refoulement (20) dans une position de commutation et qui l'ouvre dans une autre position de commutation.

9. Distributeur selon la revendication 8, **caractérisé en ce que** la soupape de distribution est une soupape de distribution à 2/2 voies (30) ou une soupape de distribution à 3/2 voies (38), située dans la conduite de refoulement (20).

10. Distributeur selon la revendication 8 ou 9, **caractérisé en ce que** la soupape de distribution (30; 38) se situe devant la soupape de refoulement (28), dans la conduite de refoulement (20).

11. Distributeur selon la revendication 8 ou 9, **caractérisé en ce que** la soupape de distribution (30; 38) se situe derrière la soupape de refoulement (28), dans la conduite de refoulement (20).

12. Distributeur selon des revendications 6 et 10, **caractérisé en ce que** dans la conduite de retour (20) est prévue une soupape de distribution à 3/2 voies (38), qui, dans une position de commutation, ouvre la conduite de refoulement (20) et ferme la conduite de retour (32) et, dans son autre position de commutation, ferme la conduite de refoulement (20) et ouvre la conduite de retour (32).
